# EUROPEAN PATENT APPLICATION

(11) **EP 3 923 299 A1**
(43) Date of publication of application: **15.12.2021**
(21) Application number: 21178243.8
(22) Date of filing: 08.06.2021
(51) Int. Cl.: G16H 80/00, G16H 30/20, G16H 30/40, A61B 34/20

(54) **SYSTEM FOR SYNCHRONIZING THE VIEWING OF AT LEAST ONE 3D MEDICAL IMAGE BETWEEN A FIRST DISPLAYING DEVICE AND AT LEAST ONE SECOND DISPLAYING DEVICE, AND METHOD THEREOF**

(30) Priority: 09.06.2020 IT 202000013780
(71) Applicant: Sentech S.r.l., 00188 Roma (RM) (IT); Russo, Giacomo Maria, 00165 Roma (IT)
(72) Inventor: RUSSO, Giacomo Maria, 00165 Roma (IT)
(74) Representative: Ferriero, Paolo

(57) **Abstract**

The present invention relates to a system for synchronizing the viewing of at least one 3D medical image between a first displaying device (11) and at least one second displaying device (21).

Said system comprises a first control unit (1) provided with said first displaying device (11) and at least a second control unit (2) provided with said second displaying device (21).

A 3D medical image is displayable through at least one view and the first control unit (1) is configured to send the 3D medical image to the second control unit (2), through a first data transfer protocol, and at least one synchronization data vector (V₁, V₂ ... V_{N}), through a second data transfer protocol, different from the first data transfer protocol, so that the view displayed on the second displaying device (21) is the same view displayed on the first displaying device (11).

The present invention also relates to a method for synchronizing the viewing of at least one 3D medical image between a first displaying device (11) and at least one second displaying device (21).

## Description

The present invention relates to a system for synchronizing the viewing of at least one 3D medical image between a first displaying device and at least one second displaying device.

In particular, the present invention relates to the structure of a system that allows an exchange of 3D medical images (which can be obtained by magnetic resonance, computed tomography or positron emission tomography) from a first healthcare worker towards at least a second healthcare worker, in which said medical images have a format such as to be displayed in the form of one or more views.

More particularly, the structure of said system is conceived to allow an exchange of medical images from a first healthcare worker to at least a second healthcare worker so that the medical images displayed on a first displaying device by said first healthcare worker are also displayed on a second displaying device by said at least one second healthcare worker, as well as preferably an exchange of medical images from said at least one second healthcare worker to said first healthcare worker so that the images displayed on said second displaying device by said at least second healthcare worker are also displayed on said first displaying device by said first healthcare worker.

Said system can be used for a teleconsultation between a first healthcare worker and at least one second healthcare worker, who are in different places. For example, teleconsultation can be used to plan a surgery and/or to support a healthcare worker during a surgery.

The system therefore allows a cooperation between the second healthcare worker and the first healthcare worker, in which said first healthcare worker is engaged to visit a patient or to operate on a patient and said second healthcare worker is remotely connected and provides assistance to said first healthcare worker.

In the following, the description will be addressed in particular to a system applied in the field of neurosurgery.

However, the same description should not be considered limited to this specific use, since the system can also be applied in fields other than neurosurgery. For example, the system can be applied during laparoscopic or vascular surgery.

### Prior art

As is known, telemedicine is a branch of telecommunications that allows healthcare worker to remotely assist a patient.

For example, through telemedicine, a surgeon can collaborate with a doctor/healthcare worker (who may have less experience or be less qualified), so that said doctor/healthcare worker is assisted by the surgeon, for example when a patient needs to be subjected to a surgical operation, without the need for the surgeon to be present in the operating room.

This type of application is also called surgical tele-cooperation.

In fact, a surgeon can remotely receive medical images referring to the patient and exchange information with the doctor/healthcare worker to plan a surgical operation and/or assist in the surgery itself.

However, to date, telemedicine techniques are limited to using known data transfer protocols, used in the context of videoconferences, in order to share images and/or data, without a doctor remotely connected interacts directly with said images and/or said data.

In the publication of Yeongho Kim et al., "Collaborative surgical simulation over the Internet" in IEEE Internet Computing, vol. 5, no. 3, pp. 65-73, May-June 2001, a system used to virtually plan a surgical operation is described.

Virtual planning can be seen by a plurality of healthcare workers.

In particular, the system is designed to allow multiple healthcare workers to be remotely connected and to one healthcare worker at a time to work on images of 3D anatomical models, while the remaining healthcare workers share the viewing of the surgical operation planning.

However, a disadvantage of this system is that the images of 3D anatomical models are not shared "in real time" as the images of 3D anatomical models depend on how often they are updated.

In particular, if a healthcare worker performs several actions on a 3D medical image, the remaining healthcare workers do not see on their displays every single action performed by that healthcare worker but only the final result due to all the actions that said healthcare worker has performed on said 3D medical image.

For this reason, said system can be used only in a phase before a surgical operation and not in other situations, such as for example in a surgical operation.

### Aim of the invention

Aim of the present invention is to overcome said disadvantage by providing a system for synchronizing the viewing of images between a first displaying device and at least a second displaying device, so as to allow the exchange of images from a first healthcare worker to at least a second healthcare worker and preferably from said at least one second healthcare worker to said first healthcare worker, significantly reducing latency times and occupying a reduced part of bandwidth.

Consequently, the system allows said second healthcare worker to assist said first healthcare worker both in the phase of preparing a surgical operation and during a surgical operation.

A further aim of the present invention is to provide a system which is easy to use and manage.

Finally, a further aim of the present invention is to provide a method for synchronizing the viewing of images between a first displaying device and at least one second displaying device.

### Object of the invention

It is object of the invention a system for synchronizing images between a first displaying device and at least a second displaying device, according to claim 1.

Further embodiments of the system are disclosed in the dependent claims.

Furthermore, it is object of the invention a method for synchronizing images between a first displaying device and at least a second displaying device, according to claim 13.

Further embodiments of the method are disclosed in the dependent claims.

### Figure list

The present invention will be now described, for illustrative, but not limitative purposes, according to its embodiment, making particular reference to the enclosed figures, wherein:

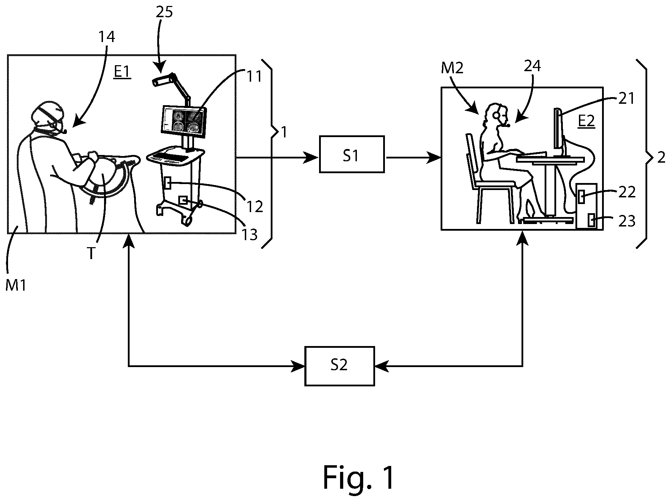

Figure 1 is a schematic view of a system for synchronizing the viewing of at least one 3D medical image according to the present invention, wherein said system comprises a first control unit and at least a second control unit, each respectively provided with a first displaying device and a second displaying device, in which said control units are configured to communicate with each other a series of data by means of a first data transfer protocol (preferably an SFTP protocol) and a further series of data by means of a second data transfer (preferably an MQTT protocol), different from the first data transfer protocol;

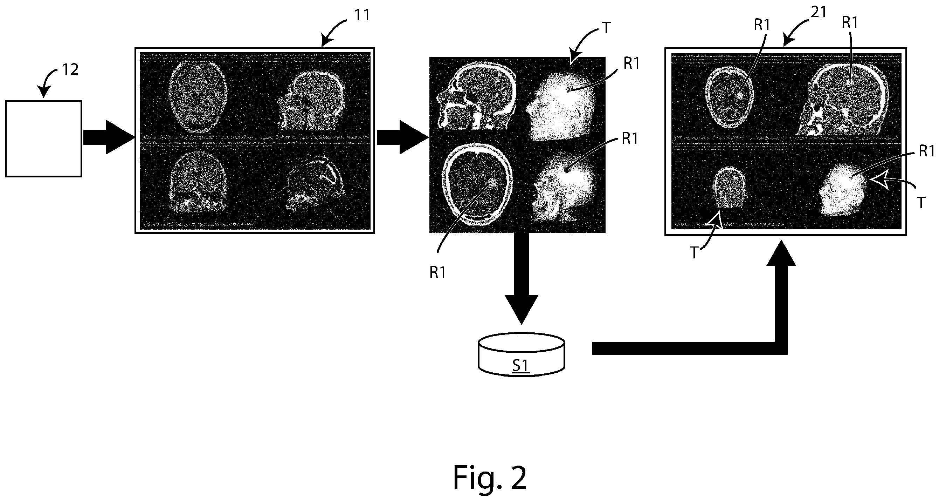

Figure 2 shows a 3D medical image stored in the first control unit and displayed on the first displaying device through a respective plurality of views, in which said 3D medical image is processed by the first control unit to identify at least a first anatomical region of interest and sent to the second control unit by means of the first data transfer protocol to be displayed on the second displaying device through said views in which said first anatomical region of interest is visible;

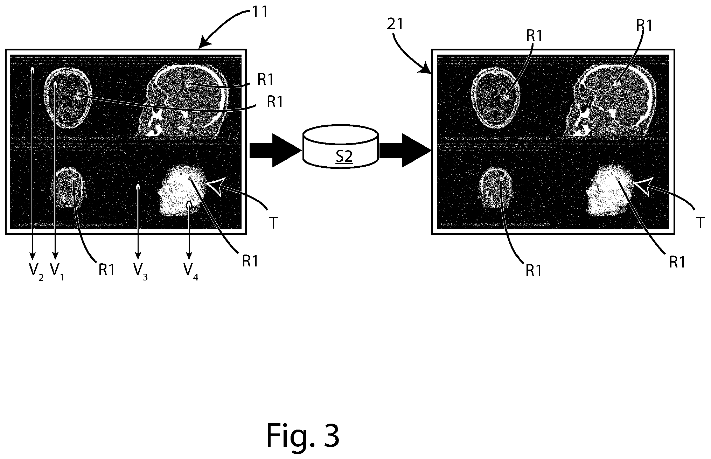

Figure 3 shows the same 3D medical image of Figure 2 displayed on the first displaying device through the respective plurality of views together with a plurality of synchronization data vectors which are generated and stored in the first control unit following an action of a first healthcare worker on at least one of said views, in which each synchronization data vector comprises a respective data group and is associated with a result due to said action, and the sending of said synchronization data vectors to the second unit control by means of the second data transfer protocol, so that the same views displayed on the first displaying device by the first healthcare worker are displayed on the second displaying device by a second healthcare worker;

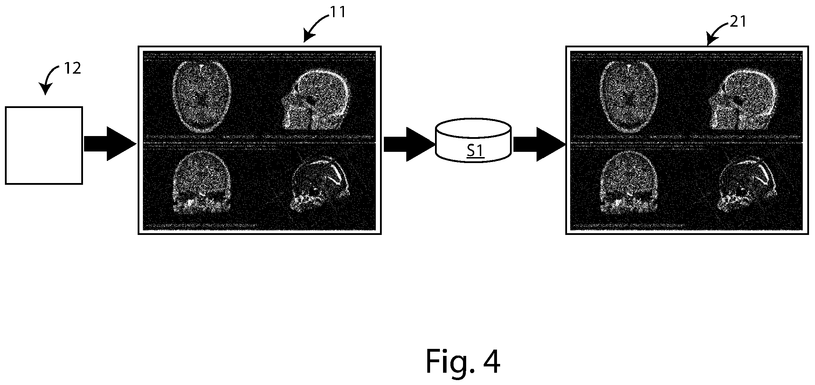

Figure 4 shows a first variant of the system in which a 3D medical image stored in the first control unit and displayed on the first displaying device through the respective plurality of views, in which said 3D medical image is sent to the second control unit by means of the first data transfer protocol, without being subjected to a processing by the first control unit to identify a first anatomical region of interest;

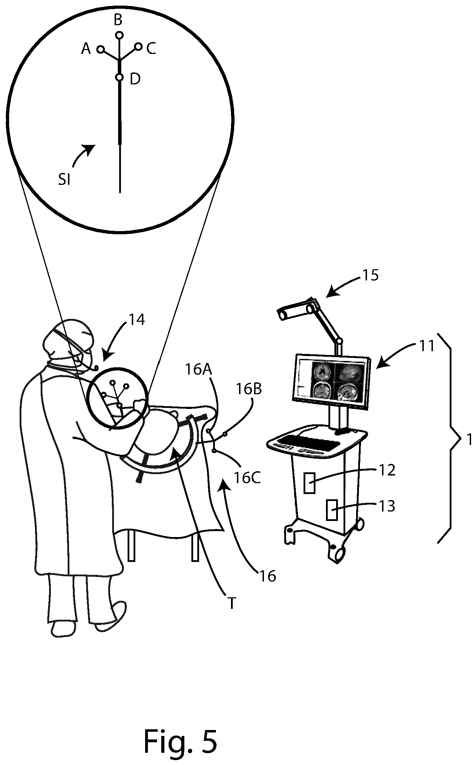

Figure 5 shows a second variant of the system, in which said system comprises a tracking device for identifying the position of a surgical instrument with respect to a patient;

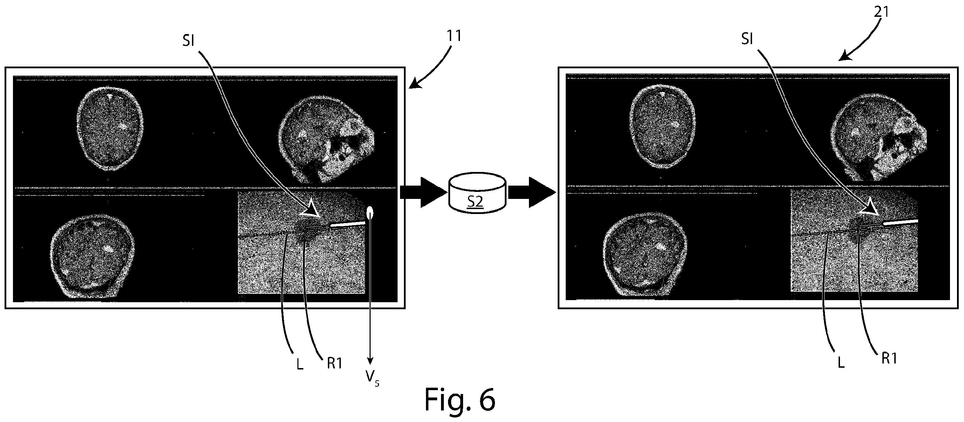

Figure 6 shows a 3D medical image displayed on the first displaying device through the respective plurality of views and a synchronization data vector comprising data associated with the position of the surgical instrument, wherein said 3D medical image and said synchronization data vector are sent to the second control unit;

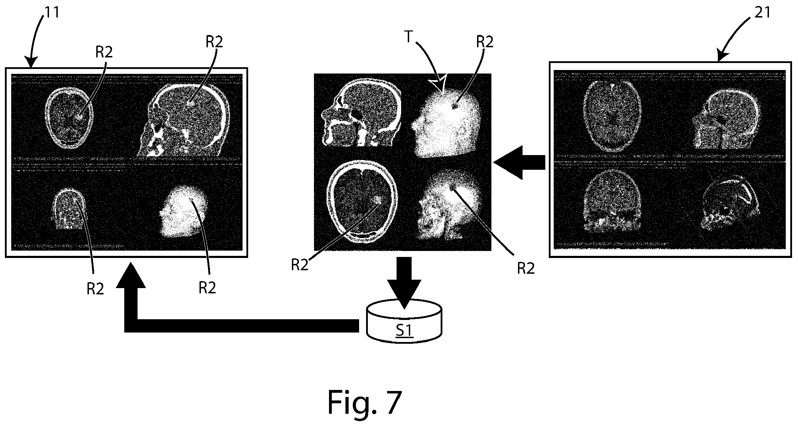

Figure 7 shows a 3D medical image (previously sent by the first control unit and received by the second control unit) displayed on the second displaying device through a plurality of views and processed by the second control unit to identify a second anatomical region of interest, in which said 3D medical image is processed to identify a second anatomical region of interest and sent to the first control unit by means of the first data transfer protocol to be displayed on the first displaying device through said views in which said second anatomical region of interest;

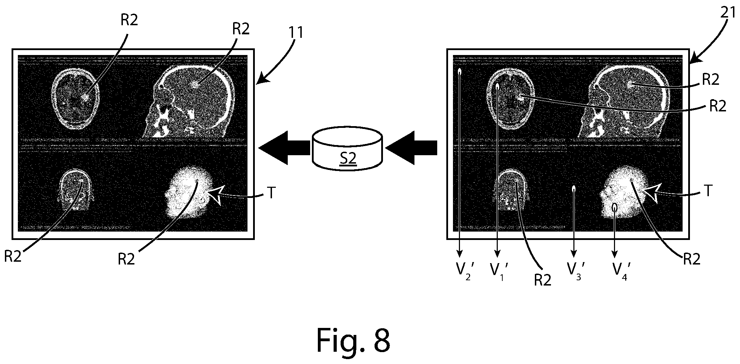

Figure 8 shows a 3D medical image displayed on the second displaying device through a respective plurality of views and a plurality of further synchronization data vectors which are generated and stored in the second control unit following an action of the second healthcare worker on at least one of said views, wherein each further synchronization data vector comprises a respective further data group and is associated with a result due to said action, and the sending of said further synchronization data vectors to the first control unit by means of the second data transfer protocol, so that the same views displayed on the second displaying device by the second healthcare worker are displayed on the first displaying device by the first healthcare worker.

### Detailed description of the invention

With particular reference to Figures 1-8, a system for synchronizing the viewing of at one or more 3D medical images between a first displaying device and at least one second displaying device is disclosed.

3D medical images can be images obtained using magnetic resonance (MRI), such as a diffusion magnetic resonance (DWI) technique, a computed tomography (CT), or a positron emission tomography (PET) imaging.

In the example being disclosed, said 3D medical images are obtained through magnetic resonance.

Furthermore, said 3D medical images are images in DICOM format.

Each 3D medical image is displayable on the first displaying device 11 (for example comprising a first display) and on the second displaying device 21 (for example comprising a second display) trough one or more views.

With particular reference to Figure 1, said system comprising a first control unit 1, to be positioned in use inside a first environment E1, and at least one second control unit 2, to be positioned in use inside a second environment E2.

The second control unit 2 is different from the first control unit 1 and the second environment E2 (inside which the second control unit 2 is positioned) is different from the first environment E1 (inside which the first control unit 1 is positioned).

The first control unit 1 is configured to communicate with the second control unit 2 and preferably the second control unit 2 is configured to communicate with the first control unit 1.

The first control unit 1 comprises said first displaying device 11 (comprising for example a first display), first storage means 12 (comprising for example a first memory) in which at least a 3D medical image is stored and a first data processing unit 13 (for example comprising a first microcontroller or a first microprocessor), connected to said first displaying device 11 and to said first storage means 12.

The second control unit 2 comprises said second displaying device 21 (comprising for example a second display), second storage means 22 (comprising for example a second memory) and a second data processing unit 23 (for example comprising a second microcontroller or a second microprocessor), connected to said second displaying device 21 and to said second storage means 22.

In the example being disclosed, inside the first environment E1 there are a first healthcare worker H1 and a patient, whose head T is visible, who is positioned on a bed (not shown), and inside the second environment E2 there is a second healthcare worker H2.

The second healthcare worker H2 is remotely connected to the first control unit 1.

Furthermore, in the example being disclosed, the system is applied in the neurosurgical field and the 3D medical images refer to the head T of the patient.

With particular reference to the first data processing unit 13, said first data processing unit 13 is configured to:
∘ send said 3D medical image to said first displaying device 11 so that at least one view is displayed (on said first displaying device 11),
∘ send said 3D medical image to said second control unit 2 through a first data transfer protocol,
∘ generate and store in said first storage means 12 one or more synchronization data vectors V₁,V₂...V_{N}, wherein each synchronization data vector V₁,V₂...V_{N} comprises a respective data group which comprises one or more data and is associated with a result due to an action of a first healthcare worker H1 on said view displayed on said first displaying device 11,
∘ send said one or more synchronization data vectors V₁,V₂...V_{N} to said second control unit 2 through a second data transfer protocol, different from said first data transfer protocol.

With particular reference to Figure 2, said 3D medical images are stored in said first storage means 12 and are acquired by said data processing unit 13 to be displayed on the first displaying device 11.

In particular, Figure 2 shows a 3D medical image referred to an anatomical part of a patient and displayed on the first display device 11 through four views. In the example described, this anatomical part is the head T of the patient.

The views shown in figure 2 are counterclockwise from the first view at the top right (following the numbering of the quadrants of a Cartesian plane) the following views:
i. a view referred to a predetermined reference plane which is a sagittal plane;
ii. a view referring to a predetermined reference plane which is a transverse plane;
iii. a view referred to a predetermined reference plane which is a coronal plane;
iv. a 3D view of a three-dimensional model.

In particular, in the 3D view of Figure 2, the three-dimensional model is obtained by combining the three predetermined reference planes shown in the other views, in which said predetermined reference planes are arranged in a Cartesian space.

Said 3D medical image is subjected to a processing by said first control unit 1 to identify at least a first anatomical region of interest R1, according to the needs of the first healthcare worker H1.

In the embodiment being disclosed, the processing performed by the first control unit 1 (i.e. by the first data processing unit 13) provides:
- a first segmentation of the 3D medical image to identify the first anatomical region of interest R1 concerning the region of the brain that will be subjected to a surgical operation, and
- a double segmentation to identify a three-dimensional model referred to the head T of the patient and a second three-dimensional model referred to the skull bones of the head T of the patient.

However, alternatively or in combination with the aforementioned segmentations, the processing may consist of generating one or more graphic markers, such as points or lines, to indicate the first anatomical region of interest R1.

During processing, the views with which the 3D medical image and the first anatomical region of interest R1 are displayed can be subjected to an action by the first healthcare worker H1. In particular, Figure 2 also shows the four views associated with the processed 3D medical image in which the first anatomical region of interest R1 is visible.

Such views are counterclockwise from the first view at the top right (following the numbering of the quadrants of a Cartesian plane) the following views:
i. a first 3D view,
ii. a view referred to a predetermined reference plane which is a sagittal plane,
iii. a view referred to a predetermined reference plane which is a transverse plane,
iv. a second 3D view.

The first 3D view shows the three-dimensional model of the head T of the patient with a first level of transparency, and the second 3D view shows the second three-dimensional model referred to the skull bones of the head T of the patient.

In particular, each three-dimensional model visible in the first 3D view and in the second 3D view is shown through a respective three-dimensional rendering.

In both 3D views the first anatomical region of interest R1 is visible in transparency.

Furthermore, said 3D medical image and the data associated with said first anatomical region of interest R1 and to said three-dimensional models are sent to the second control unit 2 by means of said first data transfer protocol, so that at least one view, in which the first anatomical region of interest R1 and preferably said three-dimensional models are visible, is displayed on the second display device 21 by the second healthcare worker H2.

In the example being described, said 3D medical image is displayed on the second display device 21 through four views together with said first anatomical region of interest R1.

Such views are counterclockwise from the first view at the top right (following the numbering of the quadrants of a Cartesian plane) the following views:
i. a view referred to a predetermined reference plane which is a sagittal plane,
ii. a view referred to a predetermined reference plane which is a transverse plane,
iii. a view referred to a predetermined reference plane which is a coronal plane,
iv. a 3D view.

A three-dimensional model of the head T of the patient is displayed in the 3D view.

In particular, said 3D medical image is sent from the first control unit (i.e. from the first data processing unit 13) to the second control unit 2 through a first data transfer protocol.

More particularly, in the example described, said first data transfer protocol is an SFTP protocol.

Alternatively, said first data transfer protocol can be a UDP, TCP, FTP protocol.

With particular reference to Figure 3, one or more synchronization data vectors V₁,V₂...V_{N} are generated by the first data processing unit 13 based on the action of the first healthcare worker H1 on at least one view displayed on the first display device 11 and stored in the first storage means 12.

For example, the group of data included in a synchronization data vector can comprise a data associated with a zoom level of the view displayed on the first display device 11 or a plurality of data associated with a position of a cursor associated with a mouse with respect to the view displayed on the first displaying device 11 or a plurality of data associated with a point drawn on the view displayed on the first displaying device 11 or a plurality of data associated with a line drawn on the view displayed on the first displaying device 11, wherein said line can be an open line or a closed line to delimit an area.

Furthermore, when the view is a 3D view, the data group can comprise a plurality of data associated with an observation point of the view displayed on the first displaying device 11 or a data associated with an opacity level of a three-dimensional model contained in the view displayed on the first displaying device 11 or, when the view is a 2D view, a data associated with a predetermined reference plane for the view displayed on the first displaying device 11.

Said synchronization data vectors V₁,V₂...V_{N} are sent from the first control unit 1 (i.e. from the first data processing unit 13) to the second control unit 2 by means of a second data transfer protocol which, as mentioned, is different from the first data transfer protocol.

In particular, in the example described, the second data transfer protocol is an MQTT protocol.

The first data transfer protocol ensures that data are exchanged securely and received correctly by the second control unit 2 and the second data transfer protocol ensures that data is exchanged quickly between the first control unit 1 and the second control unit 2, essentially in real time.

The second data processing unit 23 is configured to:
∘ receive said 3D medical image sent from said first data processing unit 13,
∘ send said 3D medical image to the second displaying device 21 so that at least one view is displayed (on said second displaying device),
∘ receive said one or more synchronization data vector V₁,V₂...V_{N} sent from said first data processing unit 13 (through said second data transfer protocol),
∘ apply the data contained in said synchronization data vectors V₁,V₂...V_{N} to said view displayed on said second displaying device 21, so that said view displayed on said second displaying device 21 is the same view displayed on said first displaying device 11.

In the example being disclosed, as shown in Figure 3, the synchronization data vectors are four:
- a first synchronization data vector V₁ referred to a view of a first slice 3D medical image along a predetermined reference plane being a transverse plane: *SliceX** SliceNumber_X;
- a second synchronization data vector V₂ referred to a zoom level of said view of said first slice of 3D medical image: *SliceXZoom** ZoomFactor_X;
- a third synchronization data vector V₃ referred to a 3D view: *Camera* *ZoomFactor * FocalPoint_X * FocalPoint_Y * FocalPoint_Z * ViewUp_X * ViewUp_Y * ViewUp_Z * Position_X * Position_Y * Position_Z;
- a fourth synchronization data vector V₄ referred to an opacity level of a three-dimensional model contained in a 3D view: *Opacity** Segm_Number * Opacity_value.

However, different synchronization data vectors can be generated and stored in addition or in alternatively to that above mentioned, without departing from the invention.

For example, it is possible to generate and store a synchronization data vector referred to a view of a second slice of 3D medical image along a predetermined reference plane which can be sagittal or coronal, or a synchronization data vector referred to a zoom level of said view of said second slice of 3D medical image.

For each synchronization data vector V₁,V₂,V₃,V₄ above mentioned, the terms separated from the symbol "*" are the data included in the respective data group of each synchronization data vector V₁,V₂,V₃,V₄.

However, a single synchronization data vector can be generated and stored from the first data processing unit 13, on the basis of the action of the first healthcare worker H1 on said 3D medical image, without departing from the invention.

The reception of the synchronization data vectors from the second control unit 2 (i.e. from the second data processing unit 23) and the application of the data contained in said synchronization data vectors to the view displayed on the second displaying device 21 allow to synchronize the views displayed on the second displaying device 21 from the second healthcare worker H2 with the views displayed on the first displaying device 11 from the first healthcare worker H1.

In other words, the second healthcare worker H2 and the first healthcare worker H1 share the same view, although said healthcare workers are positioned in different environments and the views are displayed on different displaying devices.

In this way, the second healthcare worker H2 can assist the first healthcare worker H1 while the latter visit a patient or during a chirurgical operation. The sharing of the same views allows a consult between the healthcare workers H1, H2.

Furthermore, said first data processing unit 13 can be configured to:
∘ process said 3D medical image so that a first anatomical region of interest R1 is identified within at least a view,
∘ store a plurality of data associated to said first anatomical region of interest R1,
∘ send to said first displaying device 11 said data associated to said first anatomical region of interest R1 for displaying on said first displaying device 11 said first anatomical region of interest R1 within said view, and
∘ send through said first data transfer protocol to the second control unit 2 said data associated with said first anatomical region of interest R1 to display said first anatomical region of interest R1 in said view displayed on the second displaying device 21.

In this case, at least a synchronization data vector V₁,V₂...V_{N} comprises a respective data group comprising one or more data and is associated to a result due to an action of said first healthcare worker H1 on said first view of said first anatomical region of interest R1 displayed on said first displaying device 11.

The second data processing unit 23 can be configured to:
∘ receive said data associated with said first anatomical region of interest R1,
∘ send said data associated with said first anatomical region of interest R1 to said second displaying device 21 to display said first anatomical region of interest R1 on said second displaying device 21.

In order to the first healthcare worker H1 and the second healthcare worker H2 can communicate between each other, it is preferable that said system comprises:
- a first transceiver device 14 to allow the first healthcare worker H1 to send at least one audio message to the second healthcare worker H2 and to receive at least one further audio message sent by the second healthcare worker H2, by means of said second data transfer protocol,
- a second transceiver device 24 to allow the second healthcare worker H2 to send said at least one further audio message to the first healthcare worker H1 and to receive said at least one audio message sent by the first healthcare worker H1, by means of said second data transfer protocol.

For example, each transceiver device may comprise a respective wireless headset.

The first data processing unit 13 is connected to the first transceiver device 14 and configured to:
∘ send said at least one audio message to said second control unit 2 (through said second data transfer protocol),
∘ receive said at least a further audio message sent from said second control unit 2 (through said second data transfer protocol).

The second data processing unit 23 is connected to the second transceiver device 24 and configured to:
∘ receive said at least an audio message sent from said first control unit 1 (through said second data transfer protocol),
∘ send said at least further audio message to said first control unit 1 (through said second data transfer protocol).

Figure 4 shows a first variant of the system in which a 3D medical image is stored in the first control unit 1 and displayed on the first displaying device 11 through one or more views, in the specific case a plurality of views.

However, said 3D medical image is sent from the second control unit 2 through the first data transfer protocol, without a first anatomical region of interest is identified.

As a result, the 3D medical image is not subjected to a processing performed by the first control unit 1 before said 3D medical image is sent to the second control unit 2.

In a second variant, shown in figure 5, the system comprises a tracking device for locating the position of a surgical instrument SI with respect to an anatomical part of the patient which is the head T of the patient, as said above.

The tracking device can be an optical tracking or electromagnetic device.

In the variant being disclosed, said tracking device is an optical tracking device and comprises:
- at least one stereoscopic video camera, preferably an IR stereoscopic camera, indicated with the reference number 15, and connected to the first data processing unit 13,
- a pointer 16 provided with a plurality of reflective markers 16A, 16B, 16C in which said pointer 16 is positioned at the head T of the patient and serves as a reference for the position of the surgical instrument.

Particularly, said first processing unit 13 is configured to:
∘ acquire, by means of the stereoscopic video camera 15, the position of the pointer 16, to which a reference system x, y, z is associated,
∘ store the position of said pointer 16 (i.e. the spatial coordinates),
∘ acquire, by means of the stereoscopic video camera 15, the position of said surgical instrument SI (i.e. the spatial coordinates) with respect to the reference system associated with said pointer 16,
∘ apply a transformation matrix to the reference system associated with the pointer 16 to obtain its position with respect to a reference system associated with the 3D medical image,
∘ generate and store in said first storage means 12 a synchronization data vector, the data group of which comprises a plurality of data concerning the position of said surgical instrument SI and the 3D angle with respect to the reference system associated with the 3D medical image,
∘ send a signal containing the position of said surgical instrument SI to the first displaying device 11 to display a virtual representation of said surgical instrument SI within the view displayed on the first displaying device 11,
∘ send a vector of synchronization data to the second control unit 2, by means of said second data transfer protocol, to display said surgical instrument SI within said view displayed on the second display device 21.

In particular, the transformation matrix can be calculated using known methods, in the specific case through a method called "fiduciary registration" followed by a method called "surface registration".

Fiduciary registration is a method of recording medical images based on the identification of the position of predetermined anatomical landmarks, such as nose and ears.

Surface registration is a registration method based on the overlap between a predetermined anatomical surface, such as the surface of the patient's forehead, identified by the medical image, and a surface reconstructed by the first data processing unit by interpolation of points acquired through the stereoscopic video camera, when the surgical instrument SI passes over said predetermined anatomical surface.

Furthermore, the second data processing unit 23 is configured to:
∘ receive said synchronization data vector (by means of said second data transfer protocol), and
∘ apply the data contained in said synchronization data vector to said view displayed on said second displaying device 21, so that the view displayed on said second displaying device 21 is the same view displayed on the first displaying device 11 in which said virtual representation of the surgical instrument SI is visible.

In the example being described, said surgical instrument SI is provided with a plurality of markers A, B, C, D to allow the first data processing unit 13 to acquire, by means of the stereoscopic video camera 15, the position of the surgical instrument SI with respect to the position of the pointer 16.

As shown in Figure 6, a fifth synchronization data vector V₅ is created by the first data processing unit 13 and stored in said first storage means 12: *Tool* * TipPosition_X * TipPosition_Y * TipPosition_Z * Rotation_X * Rotation_Y * Rotation_Z.

Said fifth synchronization data vector V₅ refers to the position of the surgical instrument with respect to the anatomical region of interest.

In fact, said fifth synchronization data vector V₅ comprises a group of data in turn comprising a plurality of data referred to the position and to the 3D angle of the surgical instrument S1 with respect to the individual planes identified by the associated reference system x, y, z to 3D medical image.

In this variant, the first data processing unit 13 is configured to send said fifth synchronization data vector V₅ to the second control unit 2 by means of said second data transfer protocol and the second data processing unit 23 is configured to:
∘ receive said fifth synchronization data vector V₅ (by means of said second data transfer protocol), and
∘ apply the data contained in said fifth synchronization data vector V₅ to said views displayed on said second displaying device 21, so that the views displayed on said second displaying device 21 are the same views displayed on the first displaying device 11.

Figure 6 also shows an open line L crossing the first anatomical region of interest R1, which is designed to facilitate the positioning of the surgical instrument SI on the head T of the patient. This line L is represented in the figure as a dash-point line L.

The data associated with said line L have been included in a synchronization data vector, previously exchanged between the two control units 1 and 2 by means of the second data transfer protocol.

Figure 7 shows a 3D medical image that was sent by the first control unit 1 and received by the second control unit 2.

This 3D medical image is displayed on the second displaying device 21 through one or more views, in the specific case a plurality of views.

These views are counterclockwise from the first view at the top right (following the numbering of the quadrants of a Cartesian plane) the following views:
i. a view referred to a predetermined reference plane which is a sagittal plane;
ii. a view referred to a predetermined reference plane which is a transverse plane;
iii. a view referred to a predetermined reference plane which is a coronal plane;
iv. a 3D view of a three-dimensional model.

In particular, in the 3D view of figure 7, the three-dimensional model is formed by the combination of the three predetermined reference planes shown in the other views, in which said predetermined reference planes are arranged in a Cartesian space.

Said 3D medical image is processed by the second data processing unit 23 of the second control unit 2 to identify at least a second anatomical region of interest R2.

In the example described, the processing performed by the second control unit 2 (i.e. by the second data processing unit 23) provides:
- a first segmentation of the 3D medical image to identify the second anatomical region of interest R2 concerning the region of the brain that will be subject to a surgical operation, and
- a double segmentation to identify a three-dimensional model referred to the head T of the patient and a second three-dimensional model referred to the skull bones of the head T of the patient.

However, alternatively or in combination with the aforementioned segmentations, the processing may consist of generating one or more graphic markers, such as points or lines, to indicate the second anatomical region of interest R2.

Said second data processing unit 23 can be configured to:
∘ process said 3D medical image so that a second anatomical region of interest R1 is identified within at least one view,
∘ store a plurality of data associated with said second anatomical region of interest R2,
∘ send said data associated with said second anatomical region of interest R2 to said second displaying device 21 to display said second anatomical region of interest R2 within said view on said second displaying device 21, and
∘ send by means of said first data transfer protocol to the first control unit 1 said data associated with said second anatomical region of interest R2 to display said second anatomical region of interest R2 in said view displayed on the first displaying device 11.

In other words, after identifying the second anatomical region of interest R2, the data associated with the second anatomical region of interest R2 and with said three-dimensional models are sent from the second control unit 2 to the first control unit 1 through the first data transfer protocol so that at least a view, in which the second anatomical region of interest R2 and preferable said three-dimensional models are visible, is displayed on the first displaying device 11 from the first healthcare worker H1.

The first data processing unit 13 can be configured to:
∘ receive said data associated with said anatomical region of interest R2,
∘ send said data associated with said second anatomical region of interest R2 to said first displaying device 11 for displaying on said first displaying device 11 said second anatomical region of interest R2.

Furthermore, said second data processing unit 23 is configured to:
∘ generate and store in said second storage means 22 one or more further synchronization data vectors V₁',V₂'...V_{N}', different from said one or more synchronization data vectors V₁,V₂...V_{N}, wherein each further synchronization data vector V₁',V₂'...V_{N}' comprises a respective further data group which comprises one or more data and is associated with a result due to an action of a second healthcare worker H2 on at least one view displayed on said second displaying device 21,
∘ send said one or more further synchronization data vectors V₁',V₂'...V_{N}' to said first control unit 1 through said second data transfer protocol.

Furthermore, said first data processing unit 13 is configured to:
∘ receive said one or more further synchronization data vectors V₁',V₂'...V_{N}' (through said second data transfer protocol),
∘ apply the data contained in said further synchronization data vectors V₁',V₂'...V_{N}' to said view displayed on said first displaying device 11, so that said view displayed on said first displaying device 11 is the same view displayed on said second displaying device 21.

For example, one or more further data synchronization vectors are generated by said second data processing unit 23 on the basis of an action of the second healthcare worker H2 on at least a view displayed on the second displaying device 21 and stored in said second storage means 12.

For example, said further data group can comprise a data associated with a zoom level of the view displayed on the second displaying device 21 or a plurality of data associated to a position of a cursor associated with a mouse with respect to the view displayed on the second displaying device 21 or a plurality of data associated with a point drawn on the view displayed on the second displaying device 21 or a plurality of data associated with a line drawn on the view displayed on the second displaying device 21, wherein said line can be an open line or a closed line to delimit an area.

Furthermore, when the view is a 3D view, said further data group can comprise a plurality of data associated to an observation point of the view displayed on the second displaying device 21 or a data associated to an opacity level of a three-dimensional model contained in the view on the second displaying device 21 or, when the view is a 3D view, associated to a predetermined reference plane for the view displayed on the second displaying device 21.

In the example being disclosed, said further data synchronization vectors are four:
- a further first data synchronization vector V₁' referred to a first slice of 3D medical image along a predetermined reference plane being a transverse plane: *SliceX** SliceNumber_X;
- a further second data synchronization vector V₂' referred to a zoom level of said first slice of 3D medical image: *SliceXZoom* *ZoomFactor_X;
- a further third data synchronization vector V₃' referred to a 3D view: *Camera* * ZoomFactor * FocalPoint_X * FocalPoint_Y * FocalPoint_Z * ViewUp_X * ViewUp_Y * ViewUp_Z * Position_X * Position_Y * Position_Z;
- a further fourth data synchronization vector V₄' referred to an opacity level of a three-dimensional model contained in a 3D view: *Opacity* * Segm_Number * Opacity_value.

However, different further data synchronization vectors can be generated and stored in addition or alternatively to that above mentioned, without departing from the scope of the invention.

For example, it is possible to generate and store a further a further data synchronization referred to a view of a second slice of 3D medical image along a predetermined reference plane that can be sagittal or coronal, or a data synchronization vector referred to a zoom level of said view of said second slice of 3D medical image. For each further data synchronization vectors V₁',V₂',V₃',V₄', above mentioned, the terms "*" are the data included in the respective further data group of each further data synchronization vector V₁',V₂',V₃',V₄'.

The term "action", regardless whether if performed from the first healthcare worker H1 on a view displayed on the first displaying device 11 or from the second healthcare worker H2 on a view displayed on the second displaying device 21, means for example one of the possible activities:
- performing a zoom on a view intended as to enlarge/reduce the dimensions of the 3d medical image displayed through said view,
- positioning a cursor associated to a mouse at a point of a view,
- changing a predetermined reference plane associated to a view with a further predetermined reference plane, different from said predetermined reference plane,
- changing the observation point of a 3D view,
- changing the opacity level of a 3D model shown in a 3D view.

In regards to the data transfer protocols used for exchanging data, as already said, the first data transfer protocol can be a SFTP protocol and the second data transfer protocol can be a MQTT protocol.

In particular, as shown in Figure 1, the first control unit 1 and the second control unit 2 can be connected to a first server that is a SFTP server to send/receive data through the first data transfer protocol, i.e. the SFTP protocol.

The first server S1 is external to the two control units 1, 2 and is remotely accessible by both control units.

However, the first control unit 1 can work also as a SFTP server in such a way that said second control unit 1 through the first data transfer protocol, without that the presence of said first server S1 is necessary.

Furthermore, in the example being disclosed, the first control unit 1 and the second control unit 2 are connected to a second server S2 that is a MQTT server to send/receive data through said second data transfer protocol, i.e. the MQTT protocol.

The second server S2 is a server external to the two control units 1, 2 and is remotely accessible by both the control units.

However, the first control unit 1 can also work as a MQTT server in such a way that said second control unit 2 is connected directly to the first control unit 1 by means of the second data transfer protocol, without the presence of said second server S2 is necessary.

Said system can further comprise a first video camera to record a first video, to be positioned in use inside the first environment E1.

Said first data processing unit 13 can be connected to said first video camera and configured to send at least portion of said first video to said second control unit 2 through said second data transfer protocol.

Alternatively or in addition to the first video camera, the system can comprise a second video camera to record a second video, to be positioned in use inside said second environment E2.

Said second data processing unit 23 can be connected to said second video camera and configured to send at least portion of said second video to said first control unit 1 through said second data transfer protocol.

The present invention also relates to a method for synchronizing the viewing of at least one 3D medical image between a first displaying device 11 and at least one second displaying device 21.

Said method comprises the following steps:
A) providing a first control unit 1, arranged inside a first environment E1, wherein said first control unit 1 comprises said first displaying device 11, first storage means 12 in which said 3D medical image is stored and a first data processing unit 13 connected to said first displaying device 11 and to said first storage means 12;
B) providing a second control unit 2, arranged inside a second environment E2, different from said first environment E1, wherein said second control unit 2 comprises said second displaying device 21, second storage means 22 and a second data processing unit 23 connected to said second displaying device 21 and to said second storage means 22;
C) sending by said first data processing unit 13 said 3D medical image to said first displaying device 11 so that at least one view is displayed (on said first displaying device 11);
D) sending by said first data processing unit 13 said 3D medical image to said second control unit 2 by means of a first data transfer protocol;
E) generating and storing in said first storage means 12 one or more synchronization data vectors V₁,V₂...V_{N}, in which each synchronization data vector V₁,V₂...V_{N} comprises a data group comprising in turn one or more data and is associated with a result of an action of a first healthcare worker H1 on said view displayed on said first displaying device 11;
F) sending by said first processing unit 13 said one or more synchronization data vectors V₁,V₂...V_{N} to said second control unit 2 by means of a second data transfer protocol, different from said first data transfer protocol;
G) receiving said 3D medical image by said second data processing unit 23;
H) sending said 3D medical image to said second displaying device 21 so that at least one view is displayed (on said second displaying device 21);
I) receiving from said second data processing unit 23 said one or more synchronization data vectors V₁,V₂...V_{N};
L) applying by said second data processing unit 23 the data contained in said synchronization data vectors V₁,V₂...V_{N} to said view displayed on said second displaying device 21, so that said view displayed on said second displaying device 21 is the same view displayed on said first displaying device 11.

Furthermore, said method can comprise the following steps:
M) generating and storing by said second data processing unit 23 in said second storage means 22 one or more further synchronization data vectors V₁',V₂'...V_{N}', different from said data vectors synchronization V₁,V₂...V_{N}, in which each further synchronization data vector V₁',V₂'...V_{N}' comprises a respective further data group comprising in turn one or more data and is associated with a result due to an action of a second healthcare worker H2 on said view displayed on said second displaying device 21;
N) sending by said second data processing unit 23 said one or more further synchronization data vectors V₁',V₂'...V_{N}' to said first control unit 1 by means of said second data transfer protocol;
O) receiving from said first data processing unit 13 said one or more further synchronization vectors V₁',V₂'...V_{N}' (through said second data transfer protocol);
P) applying by said first data processing unit 13 the data contained in said further synchronization data vectors V₁',V₂'...V_{N}' to said view displayed on said first displaying device 11, so that said view displayed on said first displaying device 11 is the same view displayed on said second displaying device 21.

### Advantages

Advantageously, the system object of the invention allows a first healthcare worker H1 to share at least one 3D medical image referred to a patient with at least a second healthcare worker H2, in which said first healthcare worker and said second healthcare worker are positioned in different environments, by sending synchronization data vectors referring to the actions performed by the first healthcare worker H1 on at least one view of said 3D medical image, without said at least one view being sent to the second control unit 2 with a consequent saving of bandwidth.

In other words, in order to synchronize one or more views to be displayed on the second displaying device 21 with respect to those displayed on the first displaying device 11, it is not necessary to send to the second control unit 2 a succession of views or videos for each action, but rather only a limited number of data, contained in said synchronization data vectors.

The medical image is displayed in the form of one or more views (preferably a plurality of views) by the first healthcare worker H1 by means of the first displaying device 11 and said first healthcare worker H1, can intervene on at least one view, by means of the first unit data processing 13, according to its needs before sending said 3D medical image to the second control unit 2 by means of a first data transfer protocol.

The 3D medical image is displayed in the form of a plurality of views by the second healthcare worker H2 by means of the second displaying device 21 and the second control unit 2 allows said second healthcare worker H2 to see on said second displaying device 21 the same views that the first healthcare worker H1 sees on the first displaying device 11.

This is due to the fact that the second control unit 2 receives, by means of a second data transfer protocol (different from the first data transfer protocol), from the first control unit 1 one or more synchronization data vectors referred to the data associated with one or more actions on at least one view performed by the first H1 healthcare worker.

Furthermore, the second control unit 2 can generate and store in turn further synchronization data vectors when an action is performed by the second healthcare worker H2 on the at least one view of the received 3D medical image and send said further synchronization data vectors, by means of said second data transfer protocol, to the first control unit 1 to allow the first healthcare worker H1 to see on the first displaying device 11 the same views displayed on the second displaying device 21 by the second healthcare worker, without saying at least a view is sent to the first control unit 1 with a consequent further saving of bandwidth.

The fact that the images are sent from the first healthcare worker to the second healthcare worker through a first data transfer protocol allows a secure exchange of data and the fact that the synchronization vectors (sent from the first control unit 1 to the second control unit 2) and the further synchronization vectors (sent by the second control unit 2 to the first control unit 1) are sent by means of a second data transfer protocol allowing a rapid exchange of data, substantially in real time.

The present invention has been described for illustrative, but not limitative purposes, according to its preferred embodiment, but it is to be understood that variations and/or modifications can be carried out by a skilled in the art, without departing from the scope thereof, as defined according to enclosed claims.

## Claims

1. System for synchronizing the viewing of at least one 3D medical image between a first displaying device (11) and at least one second displaying device (21), wherein said 3D medical image is displayable through one or more views, said system comprising:
- a first control unit (1), to be positioned in use inside a first environment (E1), said first control unit (1) comprising said first displaying device (11), first storage means (12) in which said 3D medical image is stored, a first data processing unit (13) connected to said first displaying device (11) and to said first storage means (12),
- a second control unit (2), to be positioned in use inside a second environment (E2), different from said first environment, said second control unit (2) comprising said second displaying device (21), second storage means (23), a second data processing unit (23) connected to said second displaying device (21) and to said second storage means (22),
wherein
said first data processing unit (13) is configured to:
∘ send said 3D medical image to said first displaying device (11) so that at least one view is displayed,
∘ send said 3D medical image to said second control unit (2) through a first data transfer protocol,
∘ generate and store in said first storage means (12) one or more synchronization data vectors (V₁,V₂...V_{N}), wherein each synchronization data vector (V₁,V₂...V_{N}) comprises a respective data group which comprises one or more data and is associated with a result due to an action of a first healthcare worker (H1) on said view displayed on said first displaying device (11),
∘ send said one or more synchronization data vectors (V₁,V₂...V_{N}) to said second control unit (2) through a second data transfer protocol, different from said first data transfer protocol;
wherein
said second data processing unit (23) is configured to:
∘ receive said 3D medical image sent from said first data processing unit (13),
∘ send said 3D medical image to said second displaying device (21) so that at least one view is displayed,
∘ receive said one or more synchronization data vector (V₁,V₂...V_{N}) sent from said first data processing unit (13),
∘ apply the data contained in said synchronization data vectors (V₁,V₂...V_{N}) to said view displayed on said second displaying device (21), so that said view displayed on said second displaying device (21) is the same view displayed on said first displaying device (11).

2. System according to the previous claim, wherein
said second data processing unit (23) is configured to:
∘ generate and store in said second storage means (22) one or more further synchronization data vectors (V₁',V₂'...V_{N}'), different from said one or more synchronization data vectors (V₁,V₂...V_{N}), wherein each further synchronization data vector (V₁',V₂'...V_{N}') comprises a respective further data group which comprises one or more data and is associated with a result due to an action of a second healthcare worker (H2) on said view displayed on said second displaying device (21),
∘ send said one or more further synchronization data vectors (V₁',V₂'...V_{N}') to said first control unit (1) through said second data transfer protocol,
wherein
said first data processing unit (13) is configured to:
- receive said one or more further synchronization data vectors (V₁',V₂'...V_{N}'),
- apply the data contained in said further synchronization data vectors (V₁',V₂'...V_{N}') to said view displayed on said first displaying device (11), so that said view displayed on said first displaying device (11) is the same view displayed on said second displaying device (21).

3. System according to any one of the previous claims, wherein said first data transfer protocol is a UDP or TCP or FTP or SFTP protocol, preferably SFTP.

4. System according to any one of the previous claims, wherein said second data transfer protocol is an MQTT protocol.

5. System according to any one of the previous claims, wherein said 3D medical image is an image in DICOM format.

6. System according to any one of the previous claims, wherein said first data processing unit (13) is configured to:
∘ process said 3D medical image so that at least a first anatomical region of interest (R1) is identified,
∘ store in said first storage means (12) a plurality of data associated with said first anatomical region of interest (R1),
∘ send said data associated with said first anatomical region of interest (R1) to said first displaying device (11) to display said first anatomical region of interest (R1) within said view on said first displaying device (11),
∘ send through said first data transfer protocol to the second control unit (2) said data associated with said first anatomical region of interest (R1) to display said first anatomical region of interest in said view displayed on the second displaying device (21),
wherein
said second data processing unit (23) is configured to:
∘ receive said data associated with said first anatomical region of interest (R1),
∘ send said data associated with said first anatomical region of interest (R1) to said second displaying device (21) to display said first anatomical region of interest (R1) on said second displaying device (21).

7. System according to any one of the previous claims, wherein
said second data processing unit (23) is configured to:
∘ process said 3D medical image so that at least a second anatomical region of interest (R2) is identified,
∘ store in said second storage means (22) a plurality of data associated with said second anatomical region of interest (R2),
∘ send said data associated with said second anatomical region of interest (R2) to said second displaying device (21) to display said second anatomical region of interest (R2) within said view on said second displaying device (21),
∘ send through said first data transfer protocol to the first control unit (1) said data associated with said second anatomical region of interest (R2) to display said second anatomical region of interest (R2) within said view,
wherein
said first data processing unit (13) is configured to:
∘ receive said data associated with said second anatomical region of interest (R2),
∘ send said data associated with said second anatomical region of interest (R2) to said first displaying device (11) to view said second anatomical region of interest (R2) on said first displaying device (11).

8. System according to any one of the previous claims, wherein
said data group comprises a data associated with a zoom level of said view displayed on said first displaying device (11),
or
said data group comprises a plurality of data associated with a position of a cursor associated with a mouse with respect to said view displayed on said first displaying device (11),
or
said data group comprises a plurality of data associated with an observation point of said view displayed on said first displaying device (11), when said view is a 3D view,
or
said data group comprises a data associated with an opacity level of a three-dimensional model contained in said view displayed on said first displaying device (11), when said view is a 3D view,
or
said data group comprises a plurality of data associated with a point drawn on said view displayed on said first displaying device (11),
or
said data group comprises a plurality of data associated with a line drawn on said view displayed on said first displaying device (11); said line being an open line or a closed line to delimit an area,
or
said data group comprises a data associated with a predetermined reference plane for said view displayed on said first displaying device (11), when said view is a 2D view.

9. System according to any one of the claims 2-8, wherein
said further data group comprises a data associated with a zoom level of said view displayed on said second displaying device (21),
or
said further data group comprises a plurality of data associated with a position of a cursor associated with a mouse with respect to said view displayed on said second displaying device (21),
or
said further data group comprises a plurality of data associated with an observation point of said view displayed on said second displaying device (21),
or
said further data group comprises a data associated with an opacity level of a three-dimensional model contained in said view displayed on said second displaying device (21), when said view is a 3D view,
or
said further data group comprises a plurality of data associated with a point drawn on said view displayed on said second displaying device (21),
or
said further data group comprises a plurality of data associated with a line drawn on said view displayed on said second displaying device (21); said line being an open line or a closed line to delimit an area,
or
said further data group comprises a data associated with a predetermined reference plane for said view displayed on said second displaying device (21), when said view is a 2D view.

10. System according to the previous claim,
wherein
said system comprises a tracking device for locating the position of a surgical instrument (SI) with respect to a patient, in which said tracking device is an optical tracking device and comprises:
- at least one stereoscopic video camera (15) connected to the first data processing unit (13),
- a pointer (16) provided with a plurality of reflective markers (16A, 16B, 16C), in which said pointer (16) serves as a reference for the position of the surgical instrument,
wherein
said first data processing unit (13) is configured to:
∘ acquire, by means of the stereoscopic video camera (15), the position of the pointer (16), a reference system x, y, z being associated with said pointer (16),
∘ store the position of said pointer (16),
∘ acquire, by means of the stereoscopic video camera (15), the position of said surgical instrument (SI) with respect to the reference system of said pointer (16),
∘ apply a transformation matrix to the reference system associated with said pointer (16) to obtain its position with respect to a reference system associated with the 3D medical image,
∘ generate and store in said first storage means 12 a synchronization data vector, the data group of which comprises a plurality of data concerning the position of said surgical instrument SI and the 3D angle with respect to the reference system associated with the 3D medical image,
∘ send a signal containing the information associated with the position of said surgical instrument (SI) to the first displaying device (11) to display a virtual representation of said surgical instrument (SI) within said view displayed on the first displaying device (11),
∘ send said synchronization data vector to the second control unit (2), by means of said second data transfer protocol, to display said surgical instrument (SI) within said view displayed on the second displaying device (21),
wherein
said second data processing unit (23) is configured to:
∘ receive said synchronization data vector by means of said second data transmission protocol, and
∘ apply the data contained in said synchronization data vector to said view displayed on said second displaying device (21), so that the view displayed on said second displaying device (21) is the same view displayed on the first displaying device (11) in which said virtual representation of said surgical instrument (SI) is visible.

11. System according to any one of the previous claims, wherein said system comprises:
a first transceiver device (14) to allow the first healthcare worker (H1) to send at least one audio message to the second healthcare worker (H2) and to receive at least one further audio message sent by the second healthcare worker (H2), by means of said second data transfer protocol, and
a second transceiver device (24) to allow the second healthcare worker (H2) to send said at least one further audio message to the first healthcare worker (H1) and to receive said at least one audio message sent by the first healthcare worker (H1), by means of said second data transfer protocol,
wherein
said first data processing unit (13) is connected to said first transceiver device (14) and configured to:
∘ send said at least one audio message to said second control unit (2),
∘ receive said at least one further audio message sent by said second control unit (2), and
wherein
said second data processing unit (23) is connected to said second transceiver device (24) and configured to:
∘ receive said at least one audio message sent by said first control unit (13), and
∘ send said at least one further audio message to said first control unit (1).

12. System according to any one of the previous claims, wherein said system comprises:
- a first video camera for recording a first video, to be positioned in use within said first environment (E1), in which said first data processing unit (13) is connected to said first video camera and configured to send at least a portion of said first video to said second control unit (2) by means of said second data transfer protocol,
and/or
- a second video camera for recording a second video, to be positioned in use within said second environment E2, in which said second data processing unit (23) is connected to said second video camera and configured to send at least a portion of said second video to said first control unit (2) by means of said second data transfer protocol.

13. Method for synchronizing the viewing of at least one 3D medical image between a first displaying device (11) and at least one second displaying device (21), wherein said 3D medical image is displayable through one or more views, said method comprising the following steps:
A) providing a first control unit (1), arranged inside a first environment (E1), said first control unit (1) comprising said first displaying device (11), first storage means (12) in which said 3D medical image is stored, a first data processing unit (13) connected to said first displaying device (11) and to said first storage means (12);
B) providing a second control unit (2), arranged inside a second environment (E2), different from said first environment (E1), said second control unit (2) comprising said second displaying device (21), second storage means (22), a second data processing unit (23) connected to said second displaying device (21) and to said second storage means (22);
C) sending by said first data processing unit (13) said 3D medical image to said first displaying device (11) so that at least one view is displayed;
D) sending by said first data processing unit (13) said 3D medical image to said second control unit (2) by means of a first data transfer protocol;
E) generating and storing in said first storage means (12) one or more synchronization data vectors (V₁,V₂...V_{N}), in which each synchronization data vector (V₁,V₂...V_{N}) comprises a data group comprising in turn one or more data and is associated with a result of an action of a first healthcare worker (H1) on said view displayed on said first displaying device (11);
F) sending by said first processing unit (13) said one or more synchronization data vectors (V₁,V₂...V_{N}) to said second control unit (2) by means of a second data transfer protocol, different from said first data transfer protocol;
G) receiving said 3D medical image by said second data processing unit (23);
H) sending said 3D medical image to said second displaying device (21) so that at least one view is displayed,
I) receiving from said second data processing unit (23) said one or more synchronization data vectors (V₁,V₂...V_{N});
L) applying by said second data processing unit (23) the data contained in said synchronization data vectors (V₁,V₂...V_{N}) to said view displayed on said second displaying device (21), so that said view displayed on said second displaying device (21) is the same view displayed on said first displaying device (11).

14. Method according to the previous claim, wherein said method comprises the following steps:
M) generating and storing by said second data processing unit (23) in said second storage means (22) one or more further synchronization data vectors (V₁',V₂'...V_{N}'), different from said data vectors synchronization (V₁,V₂...V_{N}), in which each further synchronization data vector (V₁',V₂'...V_{N}') comprises a respective further data group comprising in turn one or more data and is associated with a result due to an action of a second healthcare worker (H2) on said view displayed on said second displaying device (21),
N) sending by said second data processing unit (23) said one or more further synchronization data vectors (V₁',V₂'...V_{N}') to said first control unit (1) by means of said second data transfer protocol;
O) receiving from said first data processing unit (13) said one or more further synchronization vectors (V₁',V₂'...V_{N}');
P) applying by said first data processing unit (13) the data contained in said further synchronization data vectors (V₁',V₂'...V_{N}') to said view displayed on said first displaying device (11), so that said view displayed on said first displaying device (11) is the same view displayed on said second displaying device (21).
